# EUROPEAN PATENT SPECIFICATION

(11) **EP 2 789 351 B1**
(45) Date of publication and mention of the grant of the patent: **23.12.2015**
(21) Application number: 14164164.7
(22) Date of filing: 10.04.2014
(51) Int. Cl.: A61L 11/00, A61B 19/02

(54) **System adapted to handle chemotherapeutic drugs and waste deriving from the use thereof**
Anlage zur Behandlung chemotherapeutischer Medikamente und Abfall aus deren Anwendung
Installation pour le traitement de médicaments chimiothérapeutiques et de déchets d'une telle utilisation

(30) Priority: 10.04.2013 IT MI20130565
(43) Date of publication of application: 15.10.2014
(73) Proprietor: Mancini, Ugo, 09121 Cagliari (IT)
(72) Inventor: Mancini, Ugo, 09121 Cagliari (IT)
(74) Representative: Baroni, Matteo

(56) References cited:
- EP-A1- 0 819 437
- EP-A2- 0 995 502
- WO-A1-2007/014312
- WO-A2-2009/064538
- DE-A1- 10 250 053
- US-A- 3 994 028
- US-A1- 2006 253 297

## Description

### Field of the invention

The present invention relates to a system adapted to handle chemotherapeutic drugs and biohazardous waste deriving from the use thereof

### Background art

Toxic (pharmaceutical) waste must be treated with particular care, in order to avoid diffusing hazardous substances into the environment.

For example, manipulations of hazardous substances such as cytostatic drugs include operations for reconstituting anticancer drugs or taking drugs from ready-for-use multidose bags, which operations, in sanitary (hospitals, analysis and research laboratories), industrial, veterinary and other working environments, must be carried out in a protected environment (e.g. white chamber) under a suction hood or within an isolator, in order to protect both the operator and the surrounding environment.

Such operations involve handling waste defined as "biohazard", in that there is a risk that highly toxic and teratogenic exhalations/vapours/aerosols/powders might be diffused into the environment.

In all environments where such manipulations or under-hood activities occur, the biohazardous or toxic product being processed, at the very instant when it exits the hood, becomes a danger for the environment and for the personnel because the latter might be contaminated with splashes, drops or aerosol produced during the processing, or because it may simply slip from the operator's hand, the bag or syringe breaking against the floor and creating a dispersion resulting in environmental contamination.

Waste resulting from these processings has been catalogued, and must be collected into suitable bins, e.g. in compliance with the UN3249 standard and the B.S. 7320 1990 British Standard, which bins must also be fit for incineration, e.g. in compliance with the (European Waste Code) EWC/CER 180108 standard; when the cart is in operation, the bins are stored in the appropriate compartment.

Guidelines specifically relating to the handling of chemotherapeutic drugs provide indications about under-hood preparation, administration and disposal. As soon as the product comes out of the protected environment of the preparation hood, it becomes dangerous and a source of pollution. Unfortunately, for all steps that follow under-hood preparation, no indications or machines exist which are specifically aimed at protecting the people and the environment.

The awareness of the dangerousness of biohazardous/toxic waste and the need for definitive disposal thereof are subjects that have already been tackled, though with non-optimal results, as concerns cytostatic waste, for example. Moreover, the known solutions do not meet the regulations on the use of specific bags or containers.

Patent US-2007946283-A describes a closed, non-rigid system for treating hazardous substances, intended for hospital and industrial isolators. Such system has however been surpassed by rigid biohazardous waste holders resistant to sharp waste (needles and scalpels), which are compliant with the regulations and suitable for incineration.

Patent EP-1994900185-A describes a transportable system for hazardous waste which, however, does not include an adequate filtering system; regulation-compliant containers are not used, and sharp or pointed objects, such as needles or scalpels, are not accounted for and therefore cannot be handled correctly.

Patent US-1983514270-A describes a system for treating hazardous substances, wherein liquids and solids are inserted into a plastic bag. Just like the preceding patent, also this patent does not provide for properly handling sharp objects.

Patent US-2010788002-A describes a system for treating hazardous substances, wherein it is not possible to correctly disinfect and decontaminate the waste insertion system; in addition, the system does not allow handling and eliminating liquids.

Patent EP-2006831011-A describes a system for treating hospital waste, which does not allow sharp object to be properly handled because the waste is placed into a bag, not into a rigid container. Moreover, when the cover is opened air movement is created near the waste, thus causing the exit of pollutants (vapours and powders) which are harmful for the operator and the environment.

Workplace safety laws specify the use of specific containers for disposing of different kinds of waste in all working environments.

In situations related to the present context, all this waste that has to be disposed of by incineration may include sharp objects (needles, scalpel blades, etc.) and toxic/biohazardous waste. The containers intended for waste coming from the processing or administration of chemotherapeutic drugs must be rigid, fitted with an irreversible sealing system, and made of a material suitable for incineration.

However, according to the rules of Good Manufacturing Practice (GMP) and to the Guidelines intended for all environments where biohazard manipulations take place, there are no indications as to the use of specific machinery for preventing pollution from waste deriving from manipulations of biohazardous/toxic products, which, as they exit the hood, are carried to the places of use and then disposed of.

Apparatuses are also known, as described in WO2007014312, DE10250053, WO2009064538, EP0819437.

Said apparatuses are intended for large systems for treating organic hospital waste that cannot ensure physical isolation of chemotherapeutic drugs or waste, which are sources of pollution due to exhalations accumulating in hospital departments and preparation areas. In particular, WO2007014312 describes a machine aiming at providing a solution to the impossibility of having a network of incinerators. The machine shreds the waste with an anvil, presses it and then disinfects it, so that it can subsequently be treated as normal waste. Disinfectants are used for the waste material.

DE10250053 describes a large fixed plant that dehydrates all waste prior to sending it to incineration, so as to minimize transportation problems and improve combustion. WO2009064538 describes a large plant for sterilization of hospital waste.

EP0819437 describes a machine that deactivates, by flocculating it, waste body fluids from surgical operations. Therefore, all known solutions do not ensure irreversible waste isolation in compliance with the regulations; in addition, they are big, fixed machines operating outside hospitals/laboratories/factories.

Said GMP's and Guidelines only recommend the use of a normal cart with raised edges and a cloth for absorbing possible emissions and leaks.

### Summary of the invention

It is therefore the object of the present invention to propose a system adapted to handle chemotherapeutic drugs and waste deriving from the use thereof, which can overcome all the above-mentioned drawbacks and insufficiencies.

The system of the invention has been conceived after a careful analysis of the existing regulations concerning the handling of toxic products and of the insufficiencies of the technical solutions known in the art. Its aim is, in fact, to minimize the possibility of diffusion of toxic agents of any kind into the environment, both when in use and during handling and disposal activities.

The system of the invention complies with the regulations in force and allows cytostatic products, for example, to be handled, brought to departments or moved to other working environments without by any means representing a source of pollution. These activities can be carried out during normal working activities, such materials being moved and disposed of within an autonomous system that operates fully in a vacuum and is compatible with the principles of biohazard hoods.

Continuous suction is ensured, when in use, by two aspirators that stay always on.

Grid plates are used for treating materials ready for use, with constant suction of possible contaminants, the contaminated air being then delivered to filters. Inside the system, continuous suction creates a vacuum that ensures accumulation of all waste into a suitable container, in a negative-pressure environment (vacuum).

Constant suction creates a vacuum both on the product shelves and in the waste bin, thus isolating toxic waste. In this manner, there is no stage of operation or use of the system during which the toxic substances being transported, whether not yet used or already used, can be diffused into the outside environment.

The air aspirated by the system to create negative pressure is filtered by a Viledon® filter, then by an HEPA 14 filter, and finally by an activated-carbon filter, prior to being reintroduced into the environment, so as to ensure the utmost safety.

The specific waste to be treated may be solid, sharp (needles/scalpels) or liquid materials. In this regard, the system exploits the characteristics imposed on waste bin manufacturers by the regulations: the specified irreversible hermetic sealing and construction materials allow the whole system to be normally disinfected by using any chemicals and chlorine-based products specifically designed for deactivating chemotherapeutic products, thus ensuring constant control on the system's safety.

The system can use both mains power and autonomous power, which allows constant use of the cart.

In accordance with claim 1, the present invention relates to a waste handling system adapted to handle chemotherapeutic drugs and waste deriving from the use thereof, characterized by having a substantially closed box-like and cart-like structure comprising:
- at least one compartment adapted to receive a waste container, said waste container being adapted to contain both solid waste, including sharp objects, and liquid waste, and being removably abutted on at least one waste loading well with an aperture accessible from the outside and from the top of the system;
- at least one grid plate positioned on the top of the structure in a lateral position with respect to said waste loading well, adapted for being accessible from the outside of the system and acting as a support for material to be treated, and communicating with said at least one waste container through a drain duct;
- an air intake and filtering system communicating with said at least one waste container and said at least one grid plate, adapted to suck waste air and output purified air from the system.

It is a particular object of the present invention to provide a system adapted to handle chemotherapeutic drugs and waste deriving from the use thereof as set out in the claims, which are an integral part of the present description.

### Brief description of the drawings

Further objects and advantages of the present invention will become apparent from the following detailed description of a preferred embodiment (and variants) thereof and from the annexed drawings, which are only supplied by way of non-limiting example, wherein:

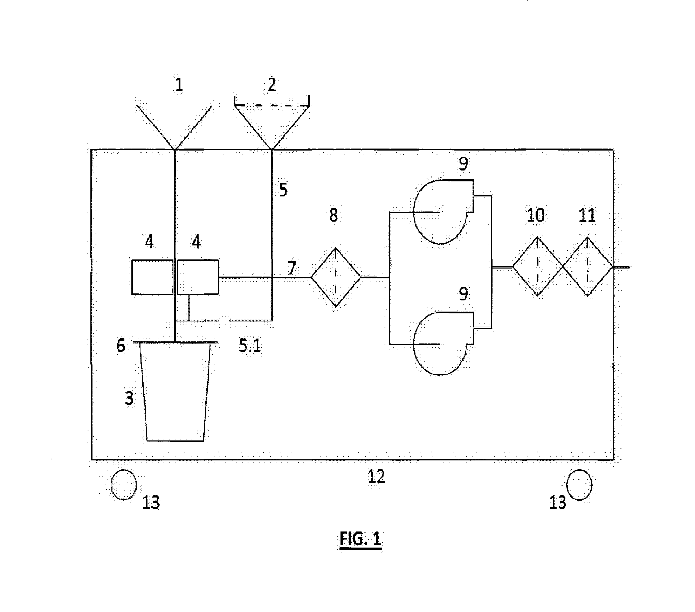

Figure 1 is a functional diagram of the parts that constitute the system according to the present invention;

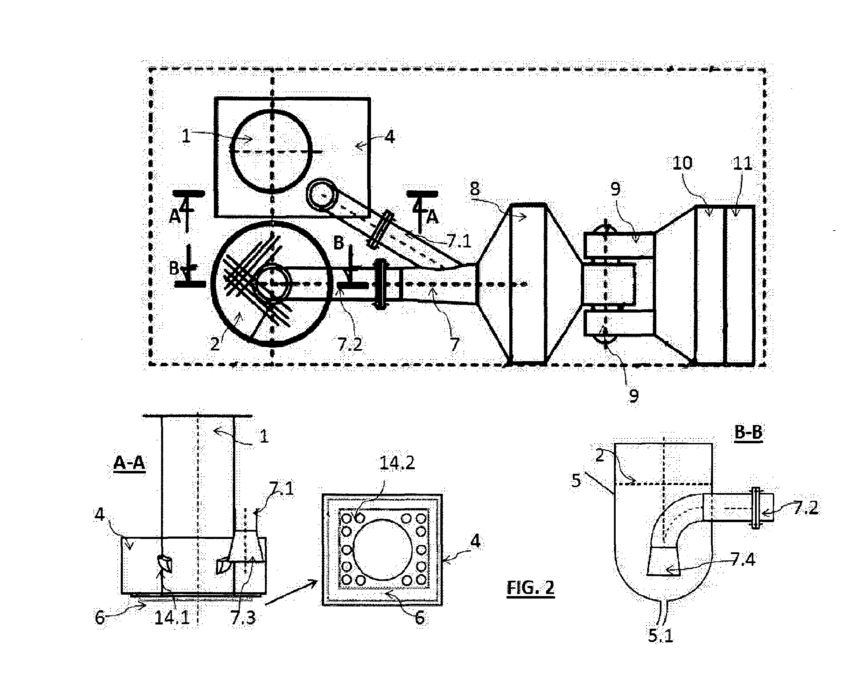

Figure 2 shows a schematic top view of the system, plus partial sectional views along the planes A-A and B-B;

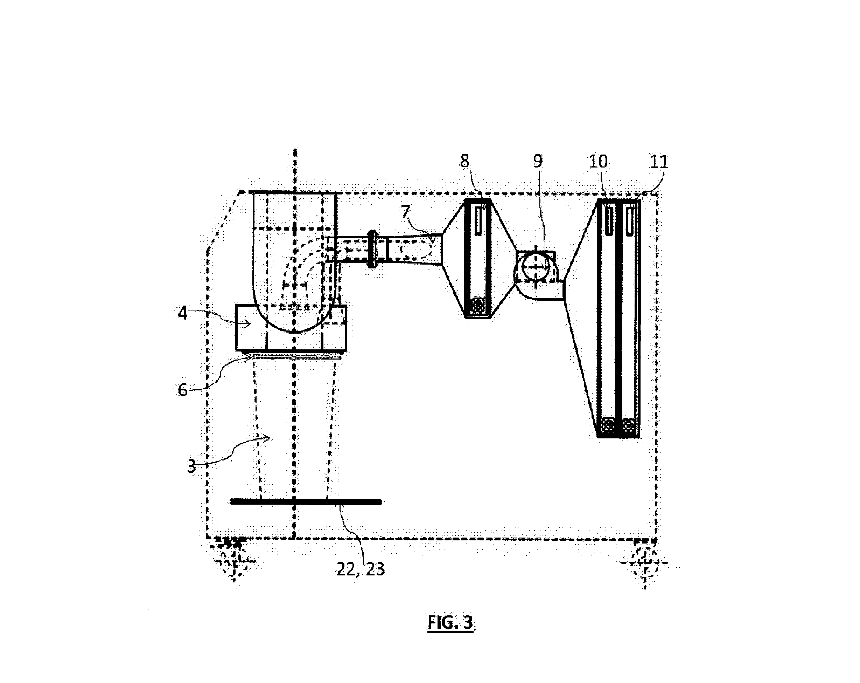

Figure 3 shows a schematic side view of the system;
Figures 4.1 to 4.4 show external side views of the cart that contains the system;
Figure 5 shows a diagram of the system for lifting and positioning the waste container in the cart.

In the drawings, the same reference numerals and letters identify the same items or components.

### Detailed description of some embodiments of the invention

With reference to the drawings, the waste handling system is preferably shaped externally like a cart enclosing the components thereof, as described below.

Inside the system there is a compartment adapted to receive a bucket-shaped waste container 3, fitted with a hermetically sealed removable cover 6. The container is removably abutted on a waste loading well 1, and is made to adhere thereto through a flange, to which a peelable gasket 6.1 is applied, after removing the cover. The well 1 is accessible from the top of the cart, for loading the material to be placed into the container, and is preferably shaped like a vertical tube.

At the junction between the loading well 1 and the waste container 3, there is a suction hood 4 having a closed box-like structure. In the hood there is an inlet 7.3 for a first branch 7.1 of the suction system. Further suction inlets are present in the area occupied by the suction hood, in particular in the side surface of the well 1 (inlets 14.1) and of the flange 6 (inlets 14.2).

A grid plate 2 is provided on the top of the cart, in a lateral position with respect to the well 1, acting as a support for the material to be treated in disinfection conditions. The grid plate 2 is put in communication with a drain duct 5 ending into a tube 5.1 that connects it to the container 3.

In the drain duct 5 there is a suction inlet 7.4 for a second branch 7.2 of the suction system, separate from the first branch 7.1.

Both branches 7.1 and 7.2 depart from the two separate suction inlets 7.3 and 7.4, and converge into an intake duct 7 of the suction system for filtering the air sucked into the two inlets.

The problem to be solved was the physical isolation of under-hood prepared drugs ready for being sent to the departments and administered, and, after the therapy, the handling of empty containers still soiled with chemotherapeutic drugs.

The system of the invention solves the problem by isolating ready-for-use drugs on the grid plate 2, where continuous suction captures and filters the exhalations produced by the chemotherapeutic drugs as they exit the preparation hood. After the therapy, the contaminated containers of the drugs just used are isolated in a technical vacuum as they are thrown into the waste container 3 (with its hood or aperture 4), which represents their final destination.

In order to avoid environmental contamination, the system of the invention is equipped with a dual open hood, wherein air travels at the velocities specified by the international regulations concerning the air curtain of biohazard hoods, e.g. 0.4 metres per second, thus solving two different problems:
- on the grid plate 2 the same velocities are attained as those of air doors of biohazard hoods (cabinets). The drugs laid on the grid plate 2 are covered by a screen, i.e. a cover with holes on its sides, so that the air constantly flowing in cannot contaminate the injectable drug, while exhalations are aspirated by the cart's constant suction system, thereby preventing environmental contamination. The cover that shields the drugs has holes on its supporting sides, and the negative pressure that prevents the dispersion of hazardous exhalations passes alongside the ready-for-use drugs without directly hitting them, thus avoiding contamination.
- access to the waste container 3 in the compartment is gained through the loading well 1, which is deliberately left without a cover: if there were a cover, in fact, it would be dangerous to touch it with soiled gloves, for both the operators themselves and the surrounding environment. The waste container 3 can therefore be accessed freely, for better hygiene and safety. Furthermore, a constant air door under negative pressure has been created for this very purpose (hood 4 with aspirator 7.3), which operates at the typical velocity of laboratory hoods while also solving another problem. According to the prior art, in fact, in order to obtain uniform air motion in a tube having a typical cross-section of such a hood (e.g. 20cm diameter), the length of the same tube should be at least equal to ten times its diameter (e.g. 2 m). On the contrary, according to one aspect of the invention, due to the elasticity of the medium (air) and to the shape of the machine, a uniform air motion in said short duct, which is only a few centimetres long, is attained by using as compensation vessels the particular shape of the hood and the arrangement of the suction holes of the hood, which stabilize the suction flow first in the hood itself and then in the waste container, which, according to the regulations, must not be filled beyond a known level, at which the suction flow can still balance itself and ensure a homogeneous air flow. The machine conceived herein can thus isolate the waste, which is introduced, under a technical vacuum with uniform motion, into the channel where waste is thrown, with no possibility of a backflow of polluted or contaminant air. In addition, due to the particular shape of the two work areas (grid plate and waste hood), disinfection and decontamination can be carried out while the machine is in operation, even by spraying or nebulizing or pouring liquids into the two work areas; the liquids will then be conveyed into the waste container. Several valves are provided in the suction system for handling situations wherein the bin is overfilled or some piece of waste obstructs the drain duct; in fact, these valves will instantaneously resume the suction flow, without creating noise or turbulences inside the work areas.

The suction system comprises two adjustable-speed suction fans 9.

There is also a first filter 8, preferably made of Viledon®, connected to the duct 7, preferably arranged in series upstream of the fans 9. Downstream of the fans there are an HEPA filter 14 and an activated-carbon filter 11, from which purified air exits and flows into the environment.

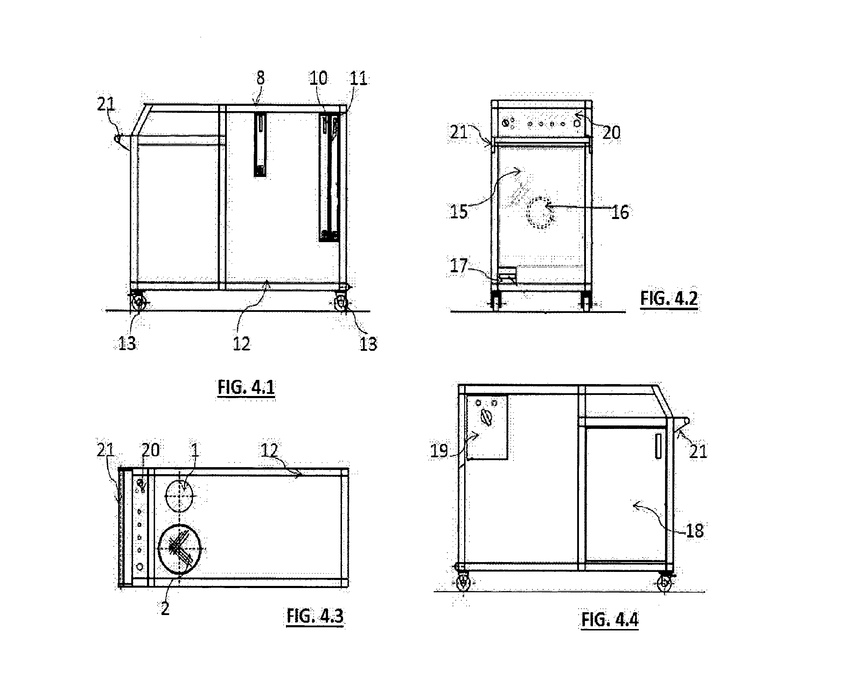

The cart (Figures 4.1 - 4.4) has a substantially closed box-like structure. The contour walls form a sealed cowling supported by pirouetting wheels 13.

On one side (Figure 4.1) there are drawers allowing access to the filters 8, 10 and 12 for maintenance purposes.

On another side (Figure 4.2) there are a control panel 20, a handle 21 for moving the cart, and a panel 15 made of transparent rigid material, e.g. LEXAN, on which there is at least one sleeve glove 16 adapted to allow the operator to insert one hand while keeping it insulated from the inside of the cart, in the compartment that houses the container 3.

On another side (Figure 4.4) there is a power panel 19 and a door 18 allowing access to the inside of the compartment that houses the container 3.

On the top side (Figure 4.3) there are the apertures for the loading well 1 and the grid plate 2.

The following will describe further details of the suction system.

Suction is generated by the two fans 9, which are preferably of the centrifugal type with appropriate flow rate and pressure head, one fan acting as a standby unit for safety. The fans are also so designed as to contribute to ensuring a noise level of less than 60 dBA outside the system.

The suction system is preferably wholly made of rigid PVC, since this material is the best in terms of resistance to corrosive substances.

The various elements of the system are preferably connected to each other by means of flanges, so that they can be fully disassembled and maintained. The junctions between the various elements are preferably created by means of gasketless flanges made of rigid PVC and compliant with the UNI standards. This solution eliminates any discontinuity or jumps between the various elements of the system.

The various sections of the tubing system are preferably sized in a manner such that the velocity of the air flowing therein will not exceed 6-8 m/s, thus reducing any hissing and rustling noise caused by the moving air.

The inlets 14.1, 14.2 in the area of the suction hood 4 are preferably so sized as to obtain constant suction along the entire well and also along the perimeter at the tube exit, as well as an air velocity of less than 3 m/s in the passage section, in order to avoid any hissing or rustling noise caused by the flowing air. Depending on the type of container, these inlets can be arranged along the drain channel (inlets 14.1) and/or in the side region of the flange on which the container rests (inlets 14.2). The suction inlets are so shaped as to not allow any liquids to enter, both during the disposal stage and during the maintenance steps required for washing the parts in contact with toxic substances. All apertures have smoothed corners, with no sharp edges, to avoid the risk of substance accumulation and to facilitate cleaning.

Below further details of the system for emitting air outside the cart are illustrated.

The aspirated air is suitably filtered and purified prior to being delivered again into the outside environment. All filters are equipped with an extraction system that allows them to be removed from the outside, so that they can be replaced when exhausted, without any risk for the operators and the environment.

As aforementioned, three filtering sections are provided:
1. A Viledon® or paper filter 8 installed downstream of the hood 4 for protecting the fans 9. This filtering septum performs the dual function of filtering coarse powders and blocking any liquids nebulized in the aspirated air.
2. An HEPA filter 14 arranged downstream of the fans 9, for blocking the emission of any particles of toxic substances into the outside environment.
3. An activated-carbon filter 11 for absorbing any odours and gaseous substances before delivering the air again into the environment.

The following will describe further details of the system for handling the waste container during the loading and unloading operations.

The compartment that houses the waste container 3 is arranged vertically under the aperture of the loading well 1, where the products to be disposed of are inserted.

Access to the waste container compartment is possible through the access door 18. During the step of loading the container 3, the whole cart has been disinfected after the previous use and the containers are new, and therefore one can access the inside of the cart without having to take any particular safety measures.

Thus, after opening the access door 18 and removing the cover of the waste container 3, the latter is positioned on a support 22, which is fitted with an automatic centering system equipped with elastic tabs, so that it can handle containers having different shapes.

The cover is hooked in a suitable position (not shown) inside the cart.

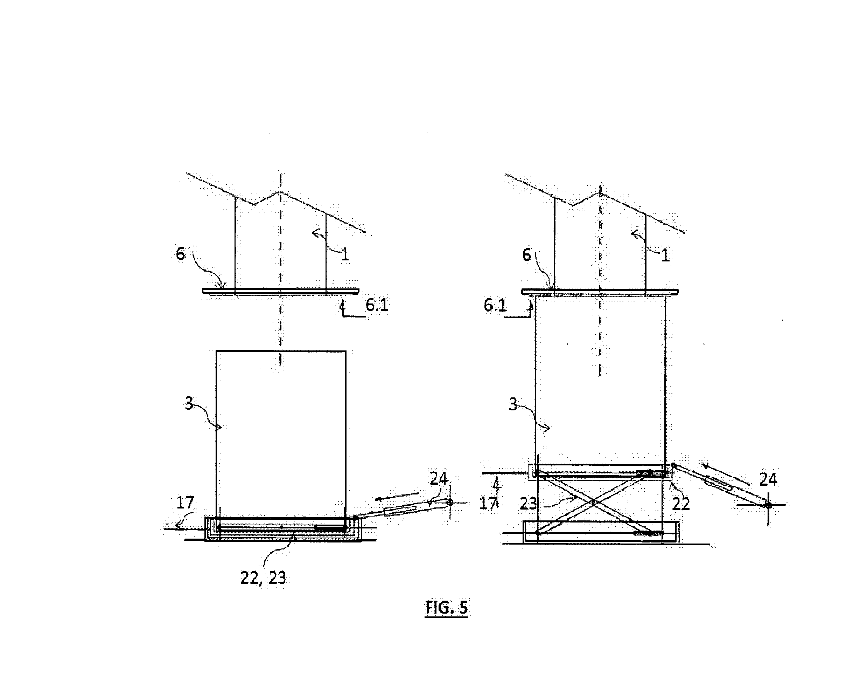

A lifting system 23 is provided for vertical positioning, preferably of the pantograph type (Figure 5). The thrust is effected, for example, by means of a gas shock absorber 24 controlled by a manual pedal 17 accessible from the outside of the cart. The pantograph system 23 lifts the container 3 and presses it against the flange 6, sealed by the peelable gasket 6.1. This ensures adherence of the container to the suction system; the adhesiveness of the gasket allows isolating the waste from the environment.

The unloading operation is very delicate, in that the container 3 will contain all the polluting substances that have been previously loaded. In order to replace the waste container, it is necessary to switch on the cart and activate a coded access procedure via the control panel 20, wherein a user code is recorded by the system.

At the beginning of the waste container replacement operations, both fans 9 are turned on, thereby increasing the suction flow. This allows the operator to replace the waste container 3 and the peelable gasket 6.1 with the utmost safety.

In order to be able to close the waste container, the latter must be disconnected from the gasket and lowered. This step is carried out by actuating the pedal 17.

Then, via the front panel 15 that allows viewing the inside of the system, and by using the sleeve glove 16, the operator takes the cover from the position where it had been hooked and closes the container in a sealed and irreversible manner.

In order to be able to open the access door 18 and extract the closed container to be replaced, one must have completed the process of hermetically sealing the container. In fact, an electronic safety locking system is active while the cart is on and until the container sealing operations have been properly carried out, when the suction system ensures disinfection.

It is therefore not possible, even intentionally, to cause contamination outside the system. The suction system can work with mains power or autonomous power, provided by means of rechargeable backup batteries.

The whole system is controlled electronically, to avoid any possibility of error by the operators. When in use, an electromagnet mounted on the door is activated to prevent accidental opening; at the end of the working day, or anyway every time the waste container needs to be changed, a safety procedure is activated, wherein the door is first unlocked and then the suction fans are turned on at a speed twice as fast as their normal suction speed, so that the waste container can be closed without any dispersion of toxic or biohazardous exhalations into the environment.

The cart's cowling and some panels can be made of AISI 304 stainless steel or carbon steel, painted with antibacterial paint. All side and top panels are made of plastic material. The cowling is mounted on four pirouetting wheels 13 made of stainless steel and fitted with polyurethane wheels suitable for white chamber use (scratch-resistant). There is also a brake pedal for stopping the cart.

Some possible variant implementations of the system may include more than one grid plate 2 for supporting the material to be treated, each with its liquid drain 5, and/or more than one waste container 3, each with its loading well 1 and suction system.

The two main stages of use of the system by operators are:
1) Handling chemotherapeutic drugs after preparation in a controlled under-hood environment, ready for transportation and use.
   This operation is carried out on one or more grid plates 2, whereon the products can be safely laid for subsequent processing, under a suitable continuous suction flow. The aspirated air is delivered directly to the filter battery.
2) Safe disposal of products after use.

One or more drain wells are provided for waste disposal: these are holes hermetically connected to one or more waste containers, into which rejects and toxic materials to be disposed of are inserted.

The rigid containers 3 (waste containers) are so manufactured as to be able to contain both solid waste, even sharp objects, and liquid waste. Waste resulting from these processings has been catalogued, and must be collected into containers suitable for this kind of disposal in compliance with the UN3249 standard and the B.S. 7320 1990 British Standard, and must be fit for incineration, e.g. in compliance with the EWC/CER (European Waste Code) 180108 standard. They must be made of rigid PVC fit for incineration, and after use they must be hermetically and irreversibly closed.

On both the grids and the wells it is also possible to vaporize, spray or pour products for disinfecting and deactivating chemotherapeutic products. Such products will then be collected, whether directly or through a condensate drain system 5, into the rigid waste containers.

## Claims

1. A waste handling system adapted to handle chemotherapeutic drugs and waste deriving from the use thereof, having a closed box and cart structure comprising:
- at least one compartment adapted to receive a waste container (3), said waste container being adapted to contain both solid waste, including sharp objects, and liquid waste, and being removably abutted on at least one waste loading well (1) with an aperture accessible from the outside and from the top of the system;
**characterized by**
- at least one grid plate (2) positioned on the top of the structure along side waste loading well (1), adapted for being accessible from the outside of the system and acting as a support for material to be treated, and communicating with said at least one waste container (3) through a drain duct (5);
- an air intake and filtering system (8-11) communicating with said at least one waste container (3) and said at least one grid plate (2), adapted to suck waste air and output purified air from the system.

2. A waste handling system according to claim 1, **characterized in that** said air intake and filtering system comprises:
- at least one suction hood (4) located at the connection between said at least one waste container (3) and said at least one waste loading well (1), said suction hood including at least one first vacuum suction inlet (7.1);
- at least one second vacuum suction inlet (7.2) in said drain duct (5);
- a suction duct (7) connected to said first and second suction inlets (7.1, 7.2);
- at least one suction fan (9) arranged in series with said suction duct (7);
- air filtering elements (8, 10, 11) arranged in series with said suction duct (7), adapted to output purified air from the system.

3. A waste handling system according to claim 1, **characterized in that** said at least one waste container (3) is removably abutted on said at least one loading well (1) through a flange (6), to which a peelable gasket (6.1) is applied.

4. A waste handling system according to claim 3, **characterized in that** it comprises further suction inlets in said suction hood (4), in the side surfaces of said waste loading well (1) and/or of the flange (6).

5. A waste handling system according to claim 2, wherein said air filtering elements comprise:
- a first filter (8) arranged upstream of and protecting said at least one suction fan (9),
- an HEPA filter (14) and an activated-carbon filter (11) arranged downstream of said at least one suction fan (9).

6. A waste handling system according to claim 3, **characterized in that** it comprises a lifting system (23) operable from the outside of the system, adapted to lift and lower said at least one waste container (3) and to hold it removably pressed against said at least one waste loading well (1) through said flange (6) and said peelable gasket (6.1).

7. A waste handling system according to claim 1, **characterized in that** said closed box structure is shaped as a cart with sealed side walls, supported by pirouetting wheels (13).

8. A waste handling system according to claim 7, **characterized in that** said side walls are fitted with:
- drawers allowing access to said filters from the outside;
- a panel (15) made of a transparent rigid material, comprising at least one sleeve glove (16) adapted to allow inserting one hand while keeping it insulated from the inside of the cart, in the area where said waste container (3) is located.

9. A waste handling system according to claim 1, **characterized in that** said at least one waste container (3) has a bucket-shape, with a removable sealing cover.

## Patentansprüche

1. Abfallhandhabungssystem zum Handhaben von Chemotherapeutika und Abfall, der von deren Verwendung erhalten wird, aufweisend eine geschlossene Gehäuse- und Wagenstruktur, umfassend:
- mindestens ein Fach, das eingerichtet ist, einen Abfallbehälter (3) aufzunehmen, wobei der Behälter eingerichtet ist, Feststoffabfall, umfassend scharfe Objekte, und flüssigen Abfall aufzunehmen und der Behälter lösbar anliegend an mindestens einem Abfalleinfüllschacht (1) mit einer Öffnung, die von der Außenseite und von der Oberseite des Systems zugänglich ist, ist;
**gekennzeichnet durch**
- mindestens eine Gitterplatte (2), die auf der Oberseite der Struktur neben dem Abfalleinfüllschacht (1) angeordnet ist, wobei die Gitterplatte eingerichtet ist, um von der Außenseite des Systems zugänglich zu sein und die Gitterplatte als Auflage für zu behandelndes Material dient, wobei die Gitterplatte (2) in Verbindung mit dem Abfallbehälter (3) **durch** einen Abzugkanal (5) steht;
- einen Lufteinlass und Filtersystem (8-11), das in Verbindung mit dem mindestens einen Abfallbehälter (3) und der mindestens einen Gitterplatte (2) steht, und eingerichtet ist, Abluft anzusaugen und gereinigte Luft vom System auszugeben.

2. Abfallhandhabungssystem nach Anspruch 1, **dadurch gekennzeichnet, dass** der Lufteinlass und das Filtersystem umfassen:
- mindestens eine Absaughaube (4), die in der Verbindung zwischen dem Abfallbehälter (3) und dem Abfalleinfüllschacht (1) angeordnet ist, wobei die Absaughaube zumindest einen Vakuumsaugeinlass (7.1) umfasst;
- mindestens einen zweiten Vakuumsaugeinlass (7.2) in dem Abzugkanal (5);
- einen Saugkanal (7), der mit dem ersten und dem zweiten Vakuumsaugeinlass (7.1, 7.2) verbunden ist;
- mindestens einen Saugventilator (9), der in Serie mit dem Saugkanal (7) angeordnet ist;
- Luftfilterelemente (8, 10, 11), die in Serie mit dem Saugkanal (7) angeordnet und eingerichtet sind, gereinigte Luft vom System auszugeben.

3. Abfallhandhabungssystem nach Anspruch 1, **dadurch gekennzeichnet, dass** der mindestens eine Abfallbehälter (3) lösbar an dem Einfüllschacht (1) durch einen Flansch (6), an den eine abziehbare Dichtung (6.1) angelegt ist, anliegt.

4. Abfallhandhabungssystem nach Anspruch 3, **dadurch gekennzeichnet, dass** es weitere Saugeinlässe in der Absaughaube (4), in den Seitenflächen des Einfüllschachts (1) und/oder des Flansches (6) umfasst.

5. Abfallhandhabungssystem nach Anspruch 2, wobei die Luftfilterelemente umfassen:
- einen ersten Filter (8), der stromaufwärts des Saugventilators (9) angeordnet ist, und den Saugventilator (9) schützt,
- einen HEPA-Filter (14) und einen Aktivkohlefilter (11), die stromabwärts vom Saugventilator (9) angeordnet sind.

6. Abfallhandhabungssystem nach Anspruch 3, **dadurch gekennzeichnet, dass** es ein Hebesystem (23) umfasst, das von der Außenseite des Systems bedienbar und eingerichtet ist, um den Abfallbehälter (3) anzuheben und abzusenken und um ihn lösbar gegen den Abfalleinfüllschacht (1) durch den Flansch (6) und die abziehbare Dichtung (6.1) gedrückt zu halten.

7. Abfallhandhabungssystem nach Anspruch 1, **dadurch gekennzeichnet, dass** die geschlossene Gehäusestruktur wie ein Wagen mit versiegelten Seitenwänden, der von drehbaren Rädern (13) gehalten wird, geformt ist.

8. Abfallhandhabungssystem nach Anspruch 7, **dadurch gekennzeichnet, dass** die Seitenwände ausgerüstet sind, mit:
- Schubfächern, die einen Zugang zu den Filtern von der Außenseite erlauben;
- eine Platte (15), die aus einem transparenten starren Material hergestellt ist und mindestens einen Ärmelhandschuh (16) umfasst, der das Einführen einer Hand erlaubt, während die Hand von der Innenseite des Wagens, in der der Abfallbehälter (3) angeordnet ist, isoliert ist.

9. Abfallhandhabungssystem nach Anspruch 1, **dadurch gekennzeichnet, dass** der mindestens eine Abfallbehälter (3) eine Eimerform mit einer abnehmbaren Versiegelungsabdeckung hat.

## Revendications

1. Système de manutention de déchets adapté pour manutentionner des médicaments et des déchets chimiothérapeutiques dérivant de leur utilisation, ayant une structure de chariot et caisse fermée comprenant :
- au moins un compartiment adapté pour recevoir un contenant à déchets (3), ledit contenant à déchets étant adapté pour contenir à la fois des déchets solides, y compris des objets pointus, et des déchets liquides, et buté de façon amovible sur au moins un puits de chargement de déchets (1) avec une ouverture accessible par l'extérieur et par le haut du système ;
**caractérisé par**
- au moins une plaque grille (2) positionnée sur le haut de la structure le long dudit puits de chargement de déchets (1), adaptée pour être accessible par l'extérieur du système et servant de support pour la matière à traiter, et communiquant avec ledit au moins un contenant à déchets (3) par l'intermédiaire d'un conduit de drainage (5) ;
- un système d'admission et de filtration d'air (8 à 11) communiquant avec ledit au moins un contenant à déchets (3) et ladite au moins une plaque grille (2), adapté pour aspirer de l'air vicié et produire en sortie de l'air purifié depuis le système.

2. Système de manutention de déchets selon la revendication 1, **caractérisé en ce que** ledit système d'admission et de filtration d'air comprend :
- au moins une hotte d'aspiration (4) située au niveau de la liaison entre ledit au moins un contenant à déchets (3) et ledit au moins un puits de chargement de déchets (1), ladite hotte d'aspiration comportant au moins une première entrée d'aspiration sous vide (7.1) ;
- au moins une seconde entrée d'aspiration sous vide (7.2) dans ledit conduit de drainage (5) ;
- un conduit d'aspiration (7) relié auxdites première et seconde entrées d'aspiration (7.1, 7.2) ;
- au moins un ventilateur d'aspiration (9) agencé en série avec ledit conduit d'aspiration (7) ;
- des éléments de filtration d'air (8, 10, 11) agencés en série avec ledit conduit d'aspiration (7) adaptés pour produire en sortie de l'air purifié depuis le système.

3. Système de manutention de déchets selon la revendication 1, **caractérisé en ce que** ledit au moins un contenant à déchets (3) bute de façon amovible sur ledit au moins un puits de chargement (1) par l'intermédiaire d'une bride (6), à laquelle un joint d'étanchéité pelable (6.1) est appliqué.

4. Système de manutention de déchets selon la revendication 3, **caractérisé en ce qu'**il comprend des entrées d'aspiration supplémentaires dans ladite hotte d'aspiration (4), dans les surfaces latérales dudit puits de chargement de déchets (1) et/ou de la bride (6).

5. Système de manutention de déchets selon la revendication 2, dans lequel lesdits éléments de filtration d'air comprennent :
- un premier filtre (8) agencé en amont de et protégeant ledit au moins un ventilateur d'aspiration (9),
- un filtre HEPA (14) et un filtre à charbon actif (11) agencés en aval dudit au moins un ventilateur d'aspiration (9).

6. Système de manutention de déchets selon la revendication 3, **caractérisé en ce qu'**il comprend un système de levage (23) pouvant être actionné depuis l'extérieur du système, adapté pour lever et abaisser ledit au moins un contenant à déchets (3) et pour le tenir appuyé de façon amovible contre ledit au moins un puits de chargement de déchets (1) par l'intermédiaire de ladite bride (6) et dudit joint d'étanchéité pelable (6.1).

7. Système de manutention de déchets selon la revendication 1, **caractérisé en ce que** ladite structure de caisse fermée est conformée comme un chariot avec des parois latérales étanches, supportée par des roues pivotantes (13).

8. Système de manutention de déchets selon la revendication 7, **caractérisé en ce que** lesdites parois latérales sont équipées :
- de tiroirs permettant un accès auxdits filtres par l'extérieur ;
- d'un panneau (15) réalisé en un matériau rigide transparent, comprenant au moins un gant à manche (16) adapté pour permettre d'insérer une main tout en la gardant isolée de l'intérieur du chariot, dans la zone où ledit contenant à déchets (3) est situé.

9. Système de manutention de déchets selon la revendication 1, **caractérisé en ce que** ledit au moins un contenant à déchets (3) a une forme de seau, avec un couvercle d'étanchéité amovible.
